# EUROPEAN PATENT APPLICATION

(11) **EP 4 563 568 A1**
(43) Date of publication of application: **04.06.2025**
(21) Application number: 23307071.3
(22) Date of filing: 28.11.2023
(51) Int. Cl.: C07C 67/08, C07C 69/18, C07C 45/79, C07C 45/82, C07C 49/17, C07C 51/44, C07C 51/47, C07C 53/08, C07D 307/58, C07D 317/20, C07D 493/20, C10L 1/185, C10L 1/19

(54) **METHOD AND INSTALLATION FOR PRODUCING FUEL ADDITIVES FROM AQUEOUS STREAM ISSUED FROM THE CONVERSION OF BIOMASS INTO OIL**

(71) Applicant: TotalEnergies OneTech, 92400 Courbevoie (FR)
(72) Inventor: Coustham, Thomas, 92400 Courbevoie (FR); Bouzouita, Donia, 92400 Courbevoie (FR); Alvarez, Pedro, 5400 Baden (CH); Delgado Dobladez, José Antonio, 28040 Madrid (ES); Águeda Maté, Vicente Ismael, 28040 Madrid (ES); Larriba Martínez, Marcos, 28040 Madrid (ES); Aranda López, Daniel, 28040 Madrid (ES); Serra Pérez, Estrella, 28040 Madrid (ES)
(74) Representative: Fédit-Loriot

(57) **Abstract**

The invention concerns a method for producing fuel additives comprising:
(a) a step of providing an aqueous stream comprising organic molecules, in particular carboxylic acids, ketones and aldehydes,
(b) an extraction step in which the aqueous stream is contacted with at least one solvent, and optionally with at least one adsorbent, and a first effluent enriched in organic molecules is recovered,
(c) a distillation step in which the first effluent is submitted to a distillation and a solvent stream comprising the solvent(s) and an organic stream comprising the organic molecules are recovered,
(e) a reacting step in which the organic stream is contacted with glycerol in the presence of an acid catalyst, to convert the organic molecules containing carboxyl groups into esters and/or the organic molecules containing carbonyl groups into acetals,
(f) a step of recovery of the effluent containing the converted organic molecules produced in step (e) as fuel additive.

## Description

### Technical domain

The invention relates to the production of fuel additives from aqueous stream issued from the conversion of biomass into oil or from the treatment of such oil. In particular, the invention concerns the valorization of aqueous fractions resulting from the production of oil by thermal liquefaction or pyrolysis of biomass or resulting from the treatment of such oil. More specifically, the present invention discloses a process and an installation for valorizing such aqueous fractions into additives useful for fuels, especially for diesel or gasoline fuels.

### Background

The treatment of biomass waste, such as lignocellulosic biomass waste, by thermal liquefaction or pyrolysis produces oils that can be valorized into biofuel. The liquid effluent obtained by these processes contain oil and an aqueous fraction. Such liquid effluent may form an emulsion or two phases depending on the process and/or conditions used. The aqueous fraction is thus not always recovered, or the liquid effluent may require further treatment such as distillation to separate the aqueous fraction from the oil. When the aqueous fraction is discarded the overall yield of the process is reduced as the aqueous fractions still contain valuable organic molecules with affinities to water due to their high oxygen content and polarity. These organic molecules include carboxylic acids, phenolic compounds, alcohols, aldehydes and ketones. In addition, oils produced by thermal liquefaction or pyrolysis may be submitted to further treatments during which we observe the formation of aqueous fractions containing some or all of the previously mentioned valuable organic molecules. Such treatments include hydrotreatment and fluidized catalytic cracking.

In the document EP3286284, the aqueous fraction of a thermal liquefaction treatment of biomass is electrolyzed to deoxygenate the organic molecules and to produce hydrocarbons, especially alkanes, that can be recycled to the thermal liquefaction treatment, thus increasing the yield of produced bio-oil.

Document WO2015/081104 discloses the recovery of organics from the aqueous phase of biomass catalytic pyrolysis and their up-grading. In the method disclosed, an aqueous stream comprising a water-soluble complex mixture of organic compounds is contacted successively with two different sorbents to recover a stream comprising light oxygenated compounds which are then converted into heavier oxygenated compounds in a reactor containing a basic catalyst. An increase of the yield of produced bio-oil can then be obtained.

Document EP3434364 discloses a method for recovering the oxygenated compounds contained in aqueous fractions derived from biomass in which the aqueous fractions are contacted with a specific catalyst under specific temperature and pressure conditions to produce mixtures of hydrocarbons and aromatic compounds by condensation, dehydration and alkylation with alcohols reactions. Alkylation with alcohols reactions are performed using alcohols already contained within the aqueous fractions treated. With the increase of the demand for fuels and additives obtained from feed of renewable origin such as biomass, there is however a need for processes of valorization of these aqueous solutions into other valuable compounds, in particular additives for fuels.

### Summary

The invention proposes a method for producing fuel additives comprising:
(a) a step of providing an aqueous stream comprising organic molecules, said organic molecules including at least carboxylic acids, ketones and aldehydes,
(b) an extraction step in which the aqueous stream provided in step (a) is contacted with at least one solvent, and optionally with at least one adsorbent, and at least a first effluent enriched in organic molecules is recovered, optionally a second effluent depleted in organic molecules is recovered,
(c) a distillation step in which the first effluent of step (b) is submitted to a distillation and at least two streams are recovered: a first solvent stream comprising the solvent and an organic stream comprising the organic molecules,
(d) an optional distillation step in which the second effluent of step (b) is submitted to a distillation step and at least two streams are recovered: a second solvent stream comprising the solvent and a second aqueous stream,
(e) a reacting step in which the organic stream of step (c) is contacted with glycerol in the presence of an acid catalyst, the organic molecules containing carboxyl groups are converted into esters by esterification and/or the organic molecules containing carbonyl groups are converted into acetals by acetalization, and an effluent containing the converted organic molecules is produced,
(f) a step of recovery of the effluent produced in step (e) as fuel additive.

The method of the invention allows recovering an effluent comprising acetals and esters obtained from glycerol, those compounds improving the cold properties and viscosity of a diesel fuel and improving the anti-knock properties as well as ignition properties of a gasoline.

In one embodiment, the aqueous stream provided in step (a) may be derived from biomass. Advantageously, such aqueous stream derived from biomass may result from production of oil(s) by pyrolysis of biomass and/or by thermal liquefaction of biomass, and/or results from upgrading treatment of biomass oil(s) obtained by pyrolysis of biomass and/or thermal liquefaction of biomass, or from upgrading treatment of liquid stream containing biomass oil and water, obtained by pyrolysis of biomass and/or thermal liquefaction of biomass. Such upgrading includes any purification and/or conversion treatment of the biomass producing an aqueous stream, such as hydrotreatment, de-oxygenation, fluidized catalytic cracking (FCC), hydrocracking, distillation or membrane purification process.

In one embodiment, the aqueous stream provided in step (a) may be selected from (i) an aqueous stream formed during pyrolysis of biomass and/or thermal liquefaction of biomass, (ii) an aqueous stream formed during upgrading of biomass oil(s) obtained by pyrolysis of biomass and/or thermal liquefaction of biomass, (iii) an aqueous stream formed during upgrading of a liquid stream containing biomass oil and water, obtained by pyrolysis of biomass and/or thermal liquefaction of biomass, (iv) two or more of the aqueous streams (i), (ii) and (iii).

In one embodiment, step (a) may include:
(a1) providing biomass,
(a2) submitting the biomass to pyrolysis or thermal liquefaction and producing a gas phase, a liquid stream containing oil and water, and a solid phase,
(a3) separating the liquid stream, and
(a4) recovering from the liquid stream an aqueous stream comprising organic molecules, said organic molecules including at least carboxylic acids, ketones and aldehydes.

Alternatively, or in combination, in one embodiment, step (a) may include:
(a5) providing biomass oil(s) or a liquid stream containing biomass oil and water, each obtained from pyrolysis of biomass and/or from thermal liquefaction of biomass,
(a6) upgrading the biomass oil(s) or the liquid stream thereby forming upgraded biomass oil(s) and an aqueous stream comprising organic molecules, said organic molecules including at least carboxylic acids, ketones and aldehydes,
(a7) recovering the aqueous stream.

Advantageously, the biomass may be selected from lignocellulosic biomass, herbaceous biomass, aquifer biomass, paper, cardboard, organic waste, or mixtures thereof.

In a first embodiment, the extraction step (b) may be an adsorption-drying- desorption process including:
(b.1) contacting the aqueous stream with at least one adsorbent adsorbing the organic molecules contained in the aqueous stream,
(b.2) drying the adsorbent,
(b.3) desorbing said at least one adsorbent using at least one solvent and recovering the first effluent enriched in organic molecules.

In a second embodiment, the extraction step (b) may be performed in a simulated moving bed device comprising at least three beds of at least one adsorbent, and includes:
(b'.1) introducing separately into the simulated moving bed device the aqueous stream and at least one solvent,
(b'.2) recovering from the simulated moving bed device the first effluent enriched in organic molecules and the second effluent depleted in organic molecules.

In the first and/or second embodiments, extraction step (b) may comprise one of the following features:
- the solvent used is selected from polar solvents, such as alcohols, ketones, ethers and/or esters, optionally from methanol, ethanol, isopropanol, n-butanol, tert-butanol, ethylene glycol, acetone, methylethylketone, dimethyl ether, ethyl tert-butyl ether (ETBE), methyl tert-butyl ether (MTBE), dimethoxymethane, methyl acetate, ethyl acetate or butyl acetate.
- the adsorbent used is selected from (i) polymers having a porous structure, (ii) zeolite-type adsorbents, (iii) clay-based adsorbents, (iv) silica-based adsorbents, (v) oxides, (vii) hydroxides, (viii) oxyhydroxides, (ix) activated carbon-based sorbents, or mixtures of at least two thereof,
- the extraction is performed at a temperature from ambient temperature to 100 °C, preferably up to 80 °C,

In a third embodiment, the extraction step (b) may be a liquid-liquid extraction step including:
(b".1) mixing the aqueous stream with at least one solvent non-miscible with water and forming an aqueous phase and an organic phase,
(b".2) separating the aqueous phase from the organic phase, where the organic phase is the first effluent enriched in organic molecules and the aqueous phase is the second effluent depleted in organic molecules.

In the third embodiment, said at least one solvent may be selected from ethers, esters, furans and aromatic hydrocarbons, optionally selected from butyl acetate, 2-Methyltetrahydrofuran, ethylacetate, methylacetate, ethylformate, methyl formate, isopropyl acetate, n-propyl acetate, ethyl tert-butyl ether, cyclopentyl-methyl ether, tert-amyl-methyl ether, benzene, toluene, xylene, ethylbenzene, phenol, aniline.

In one embodiment, distillation step (c), and optional distillation step (d), may each independently preferably be a vacuum distillation step.

in one embodiment, the first solvent stream from the distillation step (c), and optionally the second solvent stream from the optional distillation step (d), may be reused in the extraction step (b). This allows reducing the total amount of solvent used.

In one embodiment, step (e) may comprise one of the following features:
- step (e) is performed at a temperature from ambient temperature to 180 °C
- step (e) is performed at atmospheric pressure or under vacuum,
- step (e) is performed for a duration from 15 minutes to 24 hours,
- step (e) is performed while removing water formed,
- step (e) is performed using a homogeneous acid catalyst or a heterogeneous acid catalyst,
- step (e) is performed using an acid catalyst selected from sulphuric acid, 4-methylbenzenesulphonic acid, benzenesulphonic acid, methylsulphonic acid, macroporous styrene-divinylbenzene polymers, preferably comprising an acid functional group such as sulfonic acid, acidic zeolitic-based catalyst, a sulfonated zirconia, acid silica-alumina, ion exchange resins.

Another object of the invention is an installation for producing fuel additives comprising:
- an optional conversion unit for converting biomass into a gaseous phase, a liquid stream containing biomass oil and water, and a solid phase, having an inlet for receiving biomass and at least one outlet from which is recovered the liquid stream,
- an optional separation unit for separating the liquid stream into an organic phase and a first aqueous stream, having an inlet receiving the liquid stream, a first outlet from which is recovered the organic phase and a second outlet from which is recovered the first aqueous stream,
- an optional upgrading unit for upgrading the liquid stream or the organic phase into upgraded biomass oil and a second aqueous stream, having an inlet receiving the liquid stream or the organic phase, a first outlet from which is recovered the upgraded biomass oil, a second outlet from which is recovered the second aqueous stream,
- an extraction unit comprising a first inlet receiving the first and/or second aqueous stream, a second inlet receiving at least one solvent, at least one outlet from which is recovered a first effluent enriched in organic molecules, and optionally another outlet from which is recovered a second effluent depleted in organic molecules, the extraction unit optionally comprising at least one adsorbent,
- a distillation unit comprising an inlet receiving the first effluent and at least two outlets from which are recovered at least two streams: a first solvent stream comprising the solvent(s) and an organic stream comprising the organic molecules,
- an optional distillation unit comprising an inlet receiving the second effluent and at least two outlets from which are recovered at least two streams: a second solvent stream comprising the solvent(s) and a second aqueous stream,
- a reaction unit comprising an inlet receiving the organic stream and at least one outlet from which is recovered as a fuel additive an effluent produced in the reaction unit.

In one embodiment, the extraction unit may be selected from a liquid-liquid extraction column, a simulated moving bed device, and an adsorption-drying-desorption device.

### Definitions

The terms "comprising" and "includes" as used herein are synonymous with "including", "includes" or "contains", "containing", and are inclusive or open-ended and do not exclude additional features, elements or steps of unspecified methods.

The expressions % by weight and % by mass (also noted % m) have an equivalent meaning and refer to the proportion of the mass of a product relative to 100 g of a composition comprising it.

"Bio-oil" or "biomass oil" or "biocrude" means oil from the pyrolysis of biomass and/or oil from the hydrothermal liquefaction of biomass.

"Biomass pyrolysis oil" here means a crude oil resulting from the pyrolysis of biomass, optionally pretreated, for example by filtration/adsorption and/or liquid/liquid extraction. Pyrolysis is a thermochemical decomposition of biomass at high temperature in the absence of oxygen.

The term "pyrolysis" includes different modes of pyrolysis, such as, for example, fast pyrolysis, vacuum pyrolysis, catalytic pyrolysis, pyrolysis in the presence of hydrogen, and slow pyrolysis or charring, etc.

Hydrothermal liquefaction (HTL) is a thermochemical conversion process of biomass using water as a solvent, reactant and catalyst for the degradation reactions of organic matter, the water typically being in a subcritical or supercritical state.

Atmospheric pressure is the actual measured pressure. It therefore varies around the normal atmospheric pressure (1013.25 mbar).

Ambient temperature is the actual measured temperature.

The carbon, hydrogen and nitrogen content can be measured according to ASTM D5291-21.

The oxygen content can be measured according to ASTM D5622-17.

The water content can be determined according to ASTM D6304-20 by the Karl Fischer coulometric titration method using Coulomat AK. In particular, the samples to test were prepared at a concentration of 0.01 (g/l) in dry THF.

### Detailed description of the invention

### Step a) providing an aqueous stream comprising organic molecules

This step provides an aqueous stream comprising organic molecules, said organic molecules including at least carboxylic acids, ketones and aldehydes.

Typically, the aqueous stream is derived from a treatment of biomass to produce oil or from an upgrading treatment of this oil, in particular from any treatment which produces an aqueous stream, including, but not limited to, hydrotreatment, de-oxygenation, FCC (fluidized catalytic cracking), hydrocracking, distillation or membrane purification process.

In one embodiment, step (a) may include:
(a1) providing biomass,
(a2) submitting the biomass to pyrolysis or thermal liquefaction and producing a gas phase, a liquid stream containing oil and water, and a solid phase,
(a3) separating the liquid stream,
(a4) recovering from the liquid stream the aqueous stream comprising organic molecules, said organic molecules including at least carboxylic acids, ketones and aldehydes.

The biomass provided in step (a1) can be defined as an organic plant product, i.e. a product composed of or derived from agricultural or forestry plant material, and/or an organic waste including municipal solid waste, sludge from wastewater treatment plants, wood waste, agricultural waste, industrial waste.

The biomass can be selected from lignocellulosic biomass, herbaceous biomass (biomass of plants with a non-woody stem), aquifer biomass (plants growing in or under water such as algae), paper, cardboard, organic waste, alone or in mixtures.

Biomass can thus include (i) biomass produced by the surplus of agricultural land, preferably not used for human or animal food dedicated crops, called energy crops; (ii) biomass produced by deforestation (forest maintenance) or the cleaning of agricultural land; (iii) agricultural residues from crops, in particular cereal crops, vines, orchards, olive trees, fruit and vegetables including nuts, agri-food residues, (iv) forestry residues from forestry and wood processing; (v) agricultural residues from livestock farming (manure, slurry, litter, droppings,.(vi) organic waste from households (paper, cardboard, green waste, municipal solid waste,...); (vii) industrial organic waste (paper, cardboard, wood, putrescible waste,...); (viii) algal biomass, i.e. biomass formed from algae, e.g. microalgae (algal biomass can be an algal suspension obtained by harvesting algae from e.g. a bioreactor, or an algal residue obtained by dehydrating an algal suspension) or macroalgae; (ix) herbaceous biomass; (x) vegetable oils contained in certain wastes (e.g. cashew nut shells); (xi) industrial waste (B type wood); (xii) sludges from wastewater treatment plants, (xiii) digestate from anaerobic digestion plant.

Step (a2) may comprise a pyrolysis step, typically carried out at a temperature of 300-1000°C or 400-700°C, such pyrolysis being, for example, fast pyrolysis or flash pyrolysis or catalytic pyrolysis or hydropyrolysis. In particular, the pyrolysis may be a fast pyrolysis consisting of a rapid (<2 seconds) increase in temperature to 300°C-750°C, preferably 450-600°C, leading to depolymerisation and fragmentation of the biomass constituents (holocelluloses (cellulose, hemicellulose), lignin), followed by a rapid quenching of the degradation products.

Alternatively, or in combination, step (a2) may comprise a hydrothermal liquefaction (HTL) step, typically carried out at a temperature of 250-500°C and pressures of 10-50 MPa. Reaction times typically range from a few seconds to several tens of minutes.

Separation step (a3) removes the gas phase, essentially C1-C4 hydrocarbons, and the solid phase (typically coke and char) to recover only the liquid stream.

The liquid stream from step (a3) may have a significant water content (up to 95 wt% when HTL is performed and up to 60 wt% when pyrolysis is performed) which is recovered in step (a4) by methods including, but not limited to, distillation, gravity separation and centrifugation. Step (a4) may optionally be preceded by hydrotreatment or de-oxygenation when the aqueous fraction is emulsified with the organic portion (bio-oil) and/or the eventual aqueous stream formed during hydrodeoxygenation may be used in the present invention.

Step (a4) also allows recovering the biomass oil. The biomass oil recovered in step (a4) or the liquid stream recovered in step (a3) may be upgraded for purification and/or conversion, some upgrading process producing a further aqueous stream.

Thus, in one embodiment, step (a) may include:
(a5) providing biomass oil(s), or a liquid stream containing biomass oil and water, each obtained from pyrolysis of biomass and/or from thermal liquefaction of biomass,
(a6) upgrading the biomass oil(s) or the liquid stream thereby forming upgraded biomass oil(s) and an aqueous stream comprising organic molecules, said organic molecules including at least carboxylic acids, ketones and aldehydes,
(a7) recovering the aqueous stream.

The upgrading step (a6) may include any upgrading process producing upgraded biomass oil and an aqueous stream, such as, but not limited to, a hydrotreatment process, a de-oxygenation process, a FCC process, a distillation, a hydrocracking process or a membrane purification process.

Step (a7) also allows recovering the upgraded biomass oil. Step (a7) may be any appropriate separation process allowing separation of the second aqueous stream, such as, but not limited to, distillation, gravity separation and centrifugation.

The biomass oils provided in step (a5) may be provided by step (a4) previously described. The liquid stream provided in step (a5) may be provided by steps (a3), previously described.

The aqueous streams from step (a4) and/or from step (a7), obtained from the conversion of biomass and/or from the upgrading of biomass oil or liquid stream containing biomass oil, can contain 50 wt% or more of water and up to 50 wt%, of a water-soluble - organic compounds, and generally at least 5 wt% of organic compounds. This amount of organic compounds can account for up to 40 wt% of the total organics yield from the biomass.

The water-soluble organic compounds contained in the aqueous phase include phenols, alcohols catechols, aromatics, aldehydes, ketones, carboxylic acids, furans, indenols, naphthols and other oxygenated compounds. Their relative proportions increase with increasing polarity of the compound. The aqueous stream(s) derived from biomass can contain different oxygenated compounds, including alcohols, aldehydes, ketones, carboxylic acids and diacids, esters, ethers, diols, triols and polyalcohols in general, sugars, furanic derivatives, and phenolic derivatives, without being limiting examples.

The oxygenated compounds of the acid and diacid type, typically include formic acid, acetic acid, propanoic acid, butanoic acid, pentanoic acid, hexanoic acid, lactic acid, pyruvic acid, levulinic acid, tartronic acid, tartaric acid, glycolic acid, succinic acid, gluconic acid, and glucaric acid, without being limiting examples.

The oxygenated compounds of the aldehyde type, typically include formaldehyde, acetaldehyde, propanal, butanal, 2-butenal, pentanal, 2-pentenal, 3-pentenal, hexanal, 2-hexenal, 3-hexenal, 2-methyl-2-pentenal, 2-methyl-3-pentenal, 3-methyl-2-pentenal, furfural, and 5-hydroxy-methyl-furfural, without being limiting examples.

The oxygenated compounds of the ketonic type, typically include acetone, hydroxyacetone, 2-butanone, 2-pentanone, penten-2-one, 3-pentanone, penten-3-one, cyclopentanone, 2-hexanone, hexen-2-one, 3-hexanone, hexen-3-one, isophorone, vanillin, aceto-vanillin, furanone, cyclopentenone, methylcyclopentenone, butanedione, syringone, and aceto-syringone, without being limiting examples.

The aqueous stream thus recovered comprises organic molecules among which carboxylic acids, ketones and aldehydes are typically present.

### Step (b) extracting the organic molecules from the aqueous stream

In this step, the aqueous stream(s) provided in step (a) is contacted with at least one solvent, and optionally with at least one adsorbent, and at least a first effluent enriched in organic molecules is recovered, optionally a second effluent depleted in organic molecules is recovered.

This step allows reducing the water content and concentrating the organic molecules initially contained in the aqueous stream(s).

Typically, the first effluent contains at most 5 wt% of water, preferably at most 3 wt% of water, and most of the solvent(s) used for the extraction step.

Typically, when recovered, the second effluent comprises most of the water initially present in the aqueous stream(s) and traces of solvent(s) used for the extraction.

Whatever the embodiment, the volume ratio of aqueous stream(s) / solvent(s) may be from 10/90 to 90/10, from 20/80 to 80/20, from 30/70 to 70/30, from 35/65 to 65/35, from 35/65 to 60/40, or from 40/60 to 60/40, or within any of these limits.

In a first embodiment, the extraction step (b) is an adsorption-drying- desorption (ADD) process including:
(b.1) contacting the aqueous stream(s) with at least one adsorbent absorbing the organic molecules contained in the aqueous stream,
(b.2) drying the adsorbent,
(b.3) desorbing said at least one adsorbent using at least one solvent and recovering the first effluent enriched in organic molecules.

In this embodiment, the first effluent thus contains the solvent(s) used for the desorption step (b.3) as well as the organic molecules initially contained in the aqueous stream(s) which have been adsorbed on the adsorbent in step (b.1).

Step (b.1) may be performed up to the saturation of the adsorbent(s) used.

The drying of step (b.2) may be performed by heating and/or using gas. The gas used can be any gas suitable for drying, including air, preferably inert gases such as dinitrogen to limit oxidation.

The solvent(s) used in step (b.3) may be any solvent in which the organic molecules, and in particular carboxylic acids, ketones and aldehydes, are soluble. Suitable solvents include polar solvents.

« Polar solvents » include any compound comprising at least one covalent bond selected from a carbon-hydrogen bond, a carbon-halogen bond, a carbon-chalcogen bond, a carbon-nitrogen bond, and having a dipolar moment different from zero. Here, the water is not considered as a polar solvent.

Suitable solvents include alcohols, for example C1-C5 alcohols, such as methanol, ethanol, isopropanol, n-butanol, tert-butanol, or ethylene glycol.

Suitable solvents also include ketones, for example, but not limited to, acetone or methylethylketone (MEK).

Suitable solvents also include ethers, for example, but not limited to, dimethyl ether, ethyl tert-butyl ether (ETBE), methyl tert-butyl ether (MTBE), dimethoxymethane, or esters, for example, but not limited to, methyl acetate, ethyl acetate or butyl acetate.

The adsorbent used in step (b.1) may be any adsorbent capable of adsorbing the organic molecules initially contained in the aqueous stream, and in particular carboxylic acids, ketones and aldehydes.

Suitable adsorbents include (i) polymers having a porous structure, able or not of ionic exchange such as ion exchange resins, (ii) zeolite-type adsorbents, including aluminosilicates and silica, (iii) clay-based adsorbents, (iv) silica-based adsorbents, (v) oxides (such as alumina, activated alumina, bayerite, spinel), (vii) hydroxides (such as hydrotalcite), (viii) oxyhydroxides (such as boehmite), (ix) activated carbon-based sorbents, and mixtures of at least two thereof.

Suitable polymers having a porous structure include, but are not limited to, crosslinked polystyrene (such as macroporous adsorption resins, for ex. D4006, D4020, H1020, H107, Sepabeads SP207 and SP70), poly(4-vinylpyridine) polymers crosslinked with divinylbenzene, including the commercial REILLEX^{®} adsorbents such as Reillex 425, cation and anion exchange resins (such as Amberlite IR-120^{®}, Amberlyst A21^{®}).

Suitable zeolite-based adsorbents include, but are not limited to, X-Faujasite, Y-Faujasite, ZSM-5, zeolite-A, zeolite MFI, aluminosilicates containing an alkaline or alkaline-earth cation, e.g. sieves 3A, 4A, 5A, 13X, e.g. marketed under the brand name Siliporite ^{®} by Ceca, and mixtures thereof. A suitable zeolite-type adsorbent includes silicalites.

Suitable clay-based adsorbents include, but are not limited to, a clay, a crushed clay (for example kaolin, bentonite, chlorite, perovskite, smectite), organoclays, an acid treated clay such as Tonsil ^{®} from Clariant, and mixtures thereof.

Suitable silica-based adsorbents include, but are not limited to, a silica gel.

Suitable activated carbon-based adsorbents include, but are not limited to, microporous activated carbon, mesoporous activated carbon, carbon molecular sieves, carbon microbeads, carbon powder, granular activated carbon, and mixtures thereof.

Other suitable adsorbents include, but are not limited to, (i) apatite, (ii) hydroxyapatite and combinations thereof, (iii) an alumina, for example an alumina obtained by precipitation of boehmite, a calcined alumina such as Ceralox ^{®} from Sasol, (iv) boehmite, (v) bayerite, (vi) hydrotalcite, (vii) a spinel such as Pural ^{®} or Puralox from Sasol, (viii) a promoted alumina, e.g. Selexsorb ^{®} from BASF, an acid promoted alumina, a zeolite and/or metal promoted alumina such as Ni, Co, Mo or a combination of at least two of them, and mixtures of at least two thereof.

The first embodiment may be performed in an ADD unit typically comprising at least one column packed with at least one adsorbent, at least one fluid distribution device for sequential introduction into said at least one column of the feed to treat, the solvent and a gas used for drying.

In a second embodiment, the extraction step (b) is performed in a simulated moving bed device comprising at least three beds of at least one adsorbent, and includes:
(b'.1) introducing separately into the simulated moving bed device the aqueous stream(s) and at least one solvent,
(b'.2) recovering from the simulated moving bed device the first effluent enriched in organic molecules and the second effluent depleted in organic molecules.

In this embodiment, the first effluent contains the organic molecules initially contained in the aqueous stream which have been adsorbed on the adsorbent(s) of the simulated moving bed device, the solvent(s) and traces of water. The second effluent contains the water and traces of the organic molecules initially contained in the aqueous stream.

As in the first embodiment, the solvent(s) are used to desorb the organic molecules from the adsorbent(s) used. The same solvent(s) and adsorbent(s) may be used.

Extraction step (b) of first and second embodiments may be performed at a temperature from ambient temperature to 100 °C, preferably up to 80 °C or 70 °C, or within any range defined by two of these limits. By way of example, extraction step (b) of the first embodiment may be performed at a temperature from ambient temperature to 80 °C, preferably up to 40 °C, for example at 30 °C.

By way of example, extraction step (b) of the second embodiment may be performed at a temperature from 30°C to 100 °C, for example at 60 °C.

The second embodiment may be implemented in a simulated moving bed device typically comprising at least one column containing at least three beds of adsorbents, at least one fluid distribution device, typically including pumps and/or valves, for introduction into said at least one column of the feed to treat, the solvent and for extraction thereof of the solvent and the treated feed.

In a third embodiment, the extraction step (b) is a liquid-liquid extraction step including:
(b".1) mixing the aqueous stream with at least one solvent non-miscible with water and forming an aqueous phase and an organic phase,
(b".2) separating the aqueous phase from the organic phase, where the organic phase is the first effluent enriched in organic molecules and the aqueous phase is the second effluent depleted in organic molecules.

In this third embodiment, the first effluent contains the organic molecules initially contained in the aqueous stream, the solvent(s) and traces of water. The second effluent contains water, traces of the organic molecules initially contained in the aqueous stream and traces of solvent.

The solvent(s) used in step (b".1) may be any solvent in which the organic molecules, and in particular carboxylic acids, ketones and aldehydes, are soluble, and which is non-miscible with water.

Suitable solvents include ethers, esters, furanes and aromatic hydrocarbons.

Suitable ethers include, but are not limited to, ethyl tert-butyl ether (ETBE), cyclopentyl-methyl ether (CPME), tert-amyl-methyl ether (TAME).

Suitable esters include acetates and formats, such as but not limited to ethylacetate, methylacetate, ethylformate, methyl formate, isopropyl acetate, n-propyl acetate.

Suitable furans (compounds including one furan cycle) include, but are not limited to, 2-Methyltetrahydrofuran (2-MTHF).

Suitable aromatic hydrocarbons include, but are not limited to, benzene, toluene, xylene, ethylbenzene, phenol, aniline, or a mixture of these solvents.

In a preferred embodiment, ETBE, 2-MTHF and ethylacetate are used as solvents.

The separating step b".2 can be any separating step capable of separating two phases, such as, but not limited to, a gravity separation or a centrifugation. In the third embodiment, the liquid-liquid extraction step may be performed using co-current or counter current column(s).

Extraction step (b) of the third embodiment is typically performed at ambient temperature.

Typically, extraction step of any of the previously described embodiments is performed at atmospheric pressure.

### Distillation step (c) and optional distillation step (d)

The distillation allows separating the organic molecules from the solvent(s) used in the extraction step (b). It is then possible to reuse the solvent(s) separated for the extraction step.

The first effluent of step (b) is submitted to a distillation to recover at least two streams: a first solvent stream comprising the solvent(s) and an organic stream comprising the organic molecules. Preferably, only two streams are recovered.

Optionally, when a second effluent is recovered, for example when the extraction step (b) is performed according to the second embodiment or third embodiment, it may be advantageous to submit this second effluent to a distillation step (d) to recover at least two streams, preferably only two streams: a second solvent stream comprising the solvent(s) and a second aqueous stream.

The distillation may be atmospheric or vacuum distillation, preferably vacuum distillation, typically performed in a fractionation column.

The organic stream comprising the organic molecules typically contains at least 95wt%, generally at least 98wt% or 99wt% of organic molecules.

The first solvent stream or the second solvent stream typically each contains at least 90 wt% of solvent, generally at least 95wt% or even at least 99wt%.

The first solvent stream, along with the second solvent stream when present, may be reused as solvent in the extraction step (b).

### Reaction step (e)

In this step, the organic molecules recovered from the distillation step (c), and in particular ketones/aldehydes and carboxylic acids, are respectively converted in the presence of an acid catalyst and of glycerol into acetals, and more specifically into solketal (IUPAC name : 2,2-Dimethyl-1,3-dioxolan-4-yl)methanol C₆H₁₂O₃), and/or into esters, and more specifically into esters of glycerol, in particular into triacetin (IUPAC name : Propane-1,2,3-triyl triacetate of formula : C₃H₅(OCOCH₃)₃).

Generally, the effluent treated in step (e) is contacted with an acid catalyst under conditions suitable for esterification and/or acetalization.

The acid catalyst may be a homogeneous acid catalyst or a heterogeneous acid catalyst.

A suitable homogeneous acid catalyst includes sulphuric acid, 4-methylbenzenesulphonic acid, benzenesulphonic acid, methylsulphonic acid, preferably sulphuric acid.

A suitable heterogeneous acid catalyst includes acidic supported catalysts such as an acidic zeolitic-based catalyst, a sulfonated zirconia, acid silica-alumina, ion exchange resins, etc...

Suitable conditions include one or several of the following conditions:
- a temperature from ambient temperature to 180 °C, preferably from 50 to 180 °C, more preferably from 60 to 150 °C, most preferably from 60 to 120 °C or within any range defined by two of these limits,
- an atmospheric pressure or under reduced pressure, for example from -990mbarg to 10 barg, preferably at atmospheric pressure,
- a contact time of the aqueous stream with the catalyst from 15 minutes to 24 hours, preferably from 30 minutes to 12 hours, more preferably from 1 hour to 6 hours, most preferably from 1 hour to 4 hours or within any range defined by two of these limits,
- the removal, continuously or not, of the water formed by the acetalization and esterification reactions,
- any combination of the above envisaged conditions.

The removal of water during step (e) may be performed by (i) the use of a trap containing an absorbent, such as an aluminosilicate molecular sieve 4A, 13X etc..., (ii) the use of azeotropic distillation with addition of a solvent such as toluene, cyclohexane etc..., (iii) water distillation, or (iv) a combination of two or more of these methods.

During step (e), glycerol, preferably from bio-origin, is added to the organic stream from the distillation step (c).

The quantity of glycerol added to the stream is advantageously sufficient to react with aldehydes, ketones and/or with carboxylic acids present in the stream to treat. By way of example, 1 glycerol molecule for 3 carboxylic acid groups are provided for esterification, while 1 glycerol molecule for 1 aldehyde function and 1 glycerol molecule for 1 ketone function are provided. In a preferred embodiment, carboxylic acids could be separated from aldehydes and ketones to react separately. The man skilled in the art can determine the minimum quantity of glycerol to use by measuring the amount of carbonyl and carboxyl groups in the stream to treat, for example determined by carbon-13 NMR, and/or by simulation.

The effluent produced in step (e) contains the converted organic molecules from esterification and/or acetalization. As the organic stream treated in the step (e) has a high content of organic molecules (95wt% or more), a high-quality product can be obtained.

Step (e) may be performed in one or several reactors, such as, but not limited to, CSTR or a reactive distillation column.

### Recovery step (f)

The effluent produced in step (e) is typically recovered by distillation, separation, including via a membrane process or a liquid-liquid separation process, adsorption or any other suitable technique. The effluent typically contains at least 50 wt% of converted organic molecules, preferably at least 80 wt%, most preferably at least 90 wt%, most often at least 95 wt% of converted organic molecules or within any range defined by two of these limits.

The proportion of converted organic molecules (i.e. of the acetals and esters) depends on the extraction step (b) and may be tuned by selecting the extractions conditions (nature of the process, nature of the solvent(s), nature of the adsorbent(s)). Such extractions conditions can be determined by the man skilled in the art, by routine experiments and/or simulations to obtain a targeted proportion of converted organic molecules. This proportion depends also of step (e) and the its operating conditions.

### Description of the drawings

The invention will be better understood with reference to the figures, which show exemplary embodiments of the invention.
Figure 1 represents schematically an installation according to an embodiment of the invention.
Figure 2 represents schematically a part of an installation according to another embodiment of the invention.
Figure 3 represents schematically a part of an installation according to a further embodiment of the invention.
Figure 4 represents schematically the different steps of an extraction step using an adsorption - drying - desorption process.

In the figures, the same references designate the same elements.

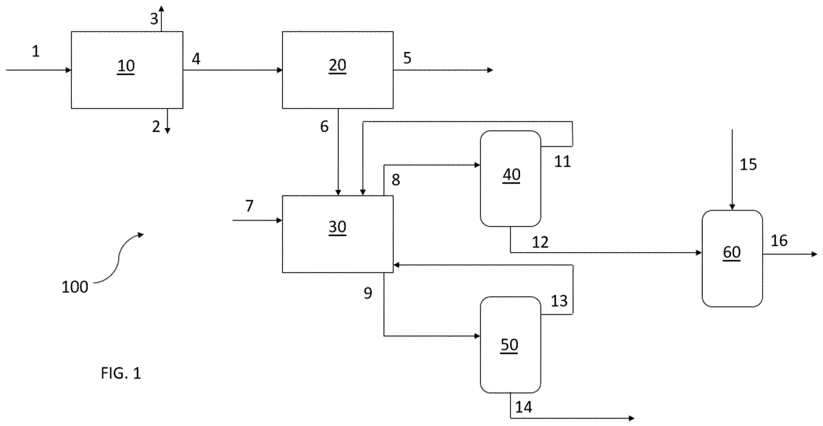

Figure 1 represent an installation 100 comprising a conversion unit 10 for converting biomass 1 into bio-oil. The conversion unit 10 may include any reactor suited for performing pyrolysis or thermal liquefaction and a separation section for separating the solid phase 2 and the gaseous phase 3 formed during the conversion. The liquid stream 4 exiting the conversion unit 10 is then sent to a treatment unit 20 which can be a separation unit for separating the liquid phase into an organic phase 5 that will be used as bio-oil and an aqueous stream 6 still containing valuable organic molecules. This separation unit is for example a fractionation unit or distillation unit, a decantation unit or a centrifugation unit. The treatment unit 20 may also be an upgrading unit generating an aqueous stream 6 and an organic phase 5. The upgrading unit may be any treatment unit such as hydrotreating unit, de-oxygenation unit, .... Several of the above-mentioned treatment units 20 may be provided. In one embodiment, a separation unit 20 may be followed by one or more upgrading units 20 or several upgrading units 20 may be provided.

This aqueous stream 6 is introduced into an extraction unit 30 in which it is contacted with at least one solvent 7, and optionally with at least one adsorbent, and a first effluent 8 enriched in organic molecules is recovered. In the embodiment represented, an optional second effluent 9 depleted in organic molecules is also recovered.

In the embodiment represented, the first effluent 8 and the second effluent 9 are sent to distillation units 40, 50, respectively, preferably vacuum distillation units. Such units 40, 50 may each comprise a distillation column, preferably a vacuum distillation column.

The distillation unit 40 receiving the first effluent 8 produces two streams: a first solvent stream 11 comprising the solvent(s) and an organic stream 12 comprising the organic molecules. Similarly, the distillation unit 50 receiving the second effluent 9 produces two streams: a second solvent stream 13 comprising the solvent(s) and an aqueous stream 14. If boiling point of molecules of interest is higher than the boiling point of solvent and if boiling point of solvent is lower than the boiling point of water, each of the solvent streams 11, 13 forming the lighter fraction of the feed are recovered as distillates, while the heavier organic or aqueous fractions are recovered as bottoms. Of course, depending on the respective boiling points of solvent, water and organic molecules of interest, the solvent streams 11 and 13 and the organic stream 12 and the aqueous stream 14 may be recovered as bottom or distillate.

The solvent streams 11 and 13 are optionally returned to the extraction unit 30 for reuse while the organic stream 12 is sent to a reaction unit 60 comprising one or several reactors in which the organic stream is contacted with glycerol 15 in the presence of an acid catalyst. In this reaction unit 60, the organic molecules containing carboxyl groups are converted into esters by esterification and/or the organic molecules containing carbonyl groups are converted into acetals by acetalization, and an effluent 16 containing the converted organic molecules is produced. The effluent 16 recovered forms a fuel additive comprising acetals and/or esters useful for diesel or gasoline range fuels. The reaction unit 60 may comprise any reactor suitable to perform the reaction.

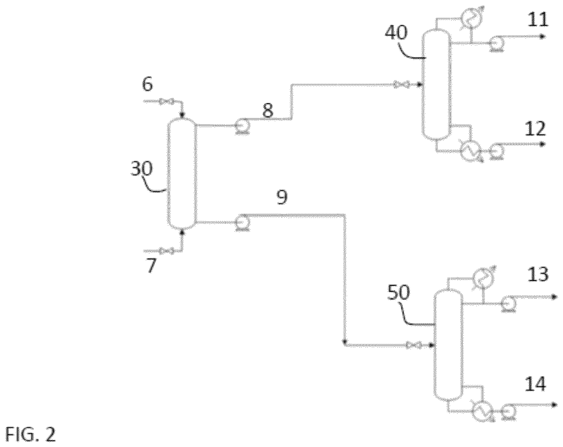

Figure 2 represents partly an installation according to the invention comprising an extraction unit 30 which is here a liquid-liquid extraction column. In this example, in which density of solvent is lower than density of aqueous phase or of organics, the solvent 7 is introduced at the bottom of the liquid-liquid extraction column 30 while the aqueous feed 6 is introduced at the top of the column. The solvent may be a single solvent or a mixture of solvents. The first effluent 8 enriched in organic molecules (here comprising mainly the solvent) is sent to a first vacuum distillation unit 40, while the second effluent 9 depleted in organic molecules (here containing mainly water) is sent to a second vacuum distillation unit 50. The light fractions 11, 13 of each distillation unit 40, 50 containing mainly solvent can then be sent back to the liquid-liquid extraction column 30 for reuse and the bottom fraction 12 of the first vacuum distillation column 40 is sent to a reaction unit 60 (not represented) as described in reference to figure 1. The bottom fraction 14 of the second vacuum distillation column 50 contains mainly water and can be further treated.

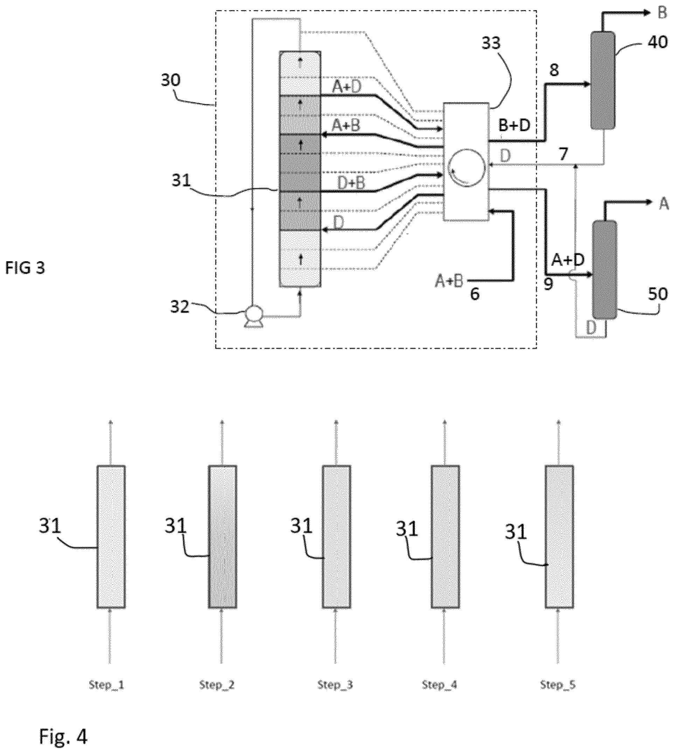

Figure 3 represents partly an installation according to an embodiment in which the extraction unit 30 comprises a SMB device having at least 3 beds of adsorbent, here 12. The extraction unit 30 thus include a column 31 containing 12 beds of adsorbent in which the circulation of the liquids is obtained via a pump 32. A rotary valve 33 assures the distribution of the incoming and exiting fluids from the column 31. The incoming fluids are the aqueous stream 6 to treat as well as the solvent 7. Two fluids exit the column 31, the first effluent 8 enriched in organic molecules and comprising the solvent, and the second effluent 9 depleted in organic molecules and comprising most of the water initially contained in the aqueous stream.

On figure 3, the aqueous stream 6 introduced into the SMB device comprises mainly water, designated by the letter A and organic molecules designated by the letter B, while D designate the solvent or the mixture of solvents used for the extraction of the organic molecules.

Figure 3 shows an ideal (perfect) separation in which introduction of fresh solvent is not required. In one embodiment not represented, fresh solvent may be introduced into the installation, for example via a line connected to the lines where the solvent D circulates or at any appropriate other point of the installation.

Two distillation units 40, 50 are also provided.

Figure 4 represents schematically the different steps of an extraction unit using an adsorption - drying - desorption process, also named ADD process.

In such an embodiment, the extraction unit 30 includes an adsorption-drying-desorption device comprising one or several columns 31 packed with at least one adsorbent, this column 31 being connected to an appropriate fluid distribution device, including one or more pump and valve, for sequential introduction into the column of the feed to treat, the solvent and a gas used for drying. The ADD process typically comprises several steps described hereafter. In a first step 1 (step_1), the solvent (or mixture of solvents) is introduced into a column containing the adsorbent to condition it. In a second step 2 (step_2), the aqueous stream is introduced into the column for adsorption of the organic molecules by the adsorbent. In a third step (step_3), the flow of aqueous stream being stopped, the column is dried, for example by air flushing. In a fourth step (step_4), a solvent (or a mixture of solvents) is introduced into the column for desorbing the organic molecules adsorbed on the adsorbent. The process can then be recommenced with a new first step. In some embodiments, depending on the nature of the adsorbent, the first step may be omitted.

### Examples

In the Examples, the complex mixtures of compounds were analyzed by GC-MS using cumene as internal standard and acetone as solvent. Four families of compounds were identified based on their chromatographic retention times. In each one of these groups, the compound in highest concentration was chosen as key component to identify the group. These key compounds' thermodynamic parameters were used in the simulation of separation processes. In the examples, the groups are named HONE, ACAC, FONE, GCOL, and their corresponding key compounds are indicated in brackets beside the names'group. The key compounds' thermodynamic parameters were used in the simulation of separation processes.

**Table 1 gives the analytic groups and their concentrations in an example of biomass oil distillate studied**

| Analytic group | Chromatographic retention time (min) | Key compound | Abbreviated name | Concentration (wt%) Biomass distillate sample |
|---|---|---|---|---|
| Group 1 | 3.53-5.85 | Hydroxyacétone | HONE | 11.47 |
| Group 2 | 5.85-7.72 | Acetic acid | ACAC | 12.39 |
| Group 3 | 7.72-10.65 | 2(5H) Furnanone | FONE | 1.67 |
| Group 4 | 10.65-13.32 | Guaiacol | GCOL | 1.17 |
| Water | | | | 73.30 |

### Example 1 : Extraction using a simulated moving bed at 30°C followed by distillation.

The extraction has been simulated using a Simulated Moving Bed (SMB) device comprising 24 beds of adsorbent.

The adsorbent used is a commercial anion exchange resin comprising poly(4-vinylpyridine) polymers crosslinked with divinylbenzene (CAS number: 9017-40-7).

The extraction test has been performed on an aqueous stream which is a model mixture of a biomass oil distillat similar to the biomass distillate sample of table 1, the composition of which is collected in table 2. The solvent used is methanol.

The simulation conditions of the extraction are the following:
- rate of introduction of the solvent into the SMB device: 0,8 kg/h,
- rate of introduction of the model mixture into the SMB device: 1 kg/h (425,1 kg/m2/h),
- rate of extraction of the raffinate: 1,039 kg/h,
- rate of extraction of the extract: 0,761 kg/h,
- Temperature inside the SMB device: 30°C
- cycle time of the model mixture inside the SMB device (i.e. duration of a cycle including all steps): 26,7 hours.

The simulated compositions of the extract and raffinate are collected into table 2. The purity and recovery yields of the organic components contained into the extract are collected in table 3.

**Table 2 Composition of the model mixture, extract and raffinate**

| | Model mixture | Raffinate | Extract |
|---|---|---|---|
| | Composition (wt %) | Composition (wt %) | Composition (wt %) |
| GCOL (Guaïacol) | 3.474 | 0.553 | 3.811 |
| FONE (2(5H)-furanone) | 3.471 | 0.007 | 4.552 |
| ACAC (acetic acid) | 9.565 | 0.000 | 12.570 |
| HONE (hydroxy acetone) | 10.191 | 0.840 | 12.250 |
| H₂O | 73.299 | 70.460 | 0.117 |
| methanol | 0.000 | 28.140 | 66.700 |

**Table 3**

| | |
|---|---|
| Organic components purities (excluding methanol) | 99,65% |
| Organic components recovery | 94,55 |
| Organic component recovery excluding GCOL | 96,21% |
| Organic component productivity | 41,76 kg/(kg of adsorbent.h) |

The vacuum distillations of each of the extract and raffinate from the extraction have then been simulated. The distillation conditions are gathered in table 4 and the composition of the distillate and bottoms obtained for each of the extract and raffinate are gathered in table 5.

**Table 4 : distillation conditions**

| | Distillation of the extract | Distillation of the raffinate |
|---|---|---|
| Pressure | 0.15 bar | 0.2 bar |
| Number of stages of the distillation column | 30 | 30 |
| Stage at which the feed is introduced | 18 | 18 |
| Reflux ratio | 0.4 | 1.15 |
| Q-reboiler | 84.4 °C | 54 °C |
| | 0.86 MJ/h | 0.85 MJ/h |

**Table 5**

| | **Distillation of the extract** | | **Distillation of the raffinate** | |
|---|---|---|---|---|
| | Distillate 1 | Bottoms 1 | Distillate 2 | Bottoms 2 |
| Pressure | 1 bar | 1 bar | 1 bar | 1 bar |
| Temperature | 22.8 °C | 84.4 °C | 22.8 °C | 54.0 °C |
| rate | 0.5076 kg/h | 0.2534 kg/h | 0.292 kg/h | 0.747 kg/h |

| **Composition (wt%)** | | | | |
|---|---|---|---|---|
| GCOL (Gaïacol) | 0.00 | 11.44 | 0.00 | 0.77 |
| FONE (2(5H)-furanone | 0.00 | 13.67 | 0.00 | 0.01 |
| ACAC (acetic acid) | 0.00 | 37.75 | 0.00 | 0.00 |
| HONE (hydroxy acetone) | 0.00 | 36.79 | 0.00 | 1.17 |
| Total Organics | 0.01 | 99.65 | 0.01 | 1.948 |
| H₂O | 0.00 | 0.34 | 0.00 | 98.05 |
| Methanol | 99.99 | 0.009 | 99.99 | 0.002 |

### Example 2 : Extraction tests by adsorption-drying-desorption followed by distillation

Two adsorption-drying-desorption extraction tests have been simulated on a mixture which composition is detailed in table 6, a first test A by using the same adsorbent as example 1 (CAS number : 9017-40-7), the other test B by using silicalite as adsorbent. Methanol was used as solvent in both tests.

The simulations were performed using the same following conditions :
- Temperature : 60°C
- solvent: methanol

**Table 6 Composition of the biomass distillate sample and the effluents of each test A, B**

| | Biomass distillate sample | Effluent A (adsorbent: anion exchange resin) | Effluent B (adsorbent: silicalite) |
|---|---|---|---|
| | Composition (wt %) | Composition (wt %) | Composition (wt %) |
| GCOL (Gaïacol) | 1.17 | 2.11 | 0.32 |
| FONE (2(5H)-furanone | 1.67 | 3.51 | 1.43 |
| ACAC (acetic acid) | 12.39 | 17.98 | 7.56 |
| HONE (hydroxy acetone) | 11.47 | 3.13 | 5.67 |
| H₂O | 73.30 | 1.85 | 2.13 |
| methanol | 0.00 | 71.42 | 82.90 |

The vacuum distillation of each effluent A and B has then been simulated. The distillation conditions are gathered in table 7 and the composition of the distillate and bottoms obtained for each of effluent A, B are gathered in table 8.

**Table 7 : distillation conditions**

| | Distillation of Effluent A | Distillation of effluent B |
|---|---|---|
| Pressure | 0.15 bar | 0.15 bar |
| Number of stages of the distillation column | 30 | 30 |
| Stage at which the feed is introduced | 15 | 15 |
| Reflux ratio | 0.4 | |
| Q-reboiler | 74.32 °C | °C |
| | 1.17 MJ/h | MJ/h |

**Table 8**

| | **Distillation of Effluent A (ads. anion exchange resin)** | | **Distillation of Effluent B (ads. silicalite)** | |
|---|---|---|---|---|
| | Distillate A | Bottoms A | Distillate B | Bottoms B |
| Pressure | 1 bar | 1 bar | 1 bar | 1 bar |
| Temperature | 23.41 °C | 74.32 °C | °C | °C |
| rate | 0.7236 kg/h | 0.7236 kg/h | kg/h | kg/h |

| **Composition (wt%)** | | | | |
|---|---|---|---|---|
| GCOL (Gaïacol) | 0.00 | 7.89 | 0.00 | 2.12 |
| FONE (2(5H)-furanone) | 0.00 | 13.13 | 0.00 | 9.51 |
| ACAC (acetic acid) | 0.16 | 66.82 | 0.00 | 50.34 |
| HONE (hydroxy acetone) | 0.00 | 11.69 | 0.00 | 37.74 |
| H₂O | 2.35 | 0.47 | 2.45 | 0.29 |
| Methanol | 97.49 | 0.00 | 97.55 | 0.00 |

### Example 3 : liquid-liquid extraction using ethylacetate followed by distillation

A liquid-liquid extraction by ethyl acetate of the biomass distillate sample has been performed followed by a simulation of a vacuum distillation.

The composition of the biomass distillate sample is presented into table 9.

The liquid-liquid extractions conditions are the following :
- extraction column (10 trays, 25 °C, 1 bar)
- solvent feed into the extraction column : 1 kg/ h
- sample feed into the extraction column: 1 kg/h
- rate of extraction of the extract from the extraction column : 1.1841 kg/h
- rate of extraction of the raffinate from the extraction column: 0.8159 kg/h

A raffinate and an extract are obtained from the liquid-liquid extraction, the compositions of which are collected into table 9.

**Table 9**

| | Biomass distillate Sample | Raffinate | Extract |
|---|---|---|---|
| | Composition (wt %) | Composition (wt %) | Composition (wt %) |
| GCOL (Gaïacol) | 1.17 | 0.00 | 0.99 |
| FONE (2(5H)-furanone) | 1.67 | 0.00 | 1.41 |
| ACAC (acetic acid) | 12.39 | 0.00 | 10.46 |
| HONE (hydroxy acetone) | 11.47 | 2.85 | 7.73 |
| H₂O | 73.30 | 83.32 | 4.49 |
| Ethyl acetate | 0 | 13.84 | 74.90 |

The distillation conditions of the extract and raffinate are collected into table 10 to recover a distillate and bottoms for each of the extract and the raffinate. The conditions and composition of each bottom and distillate are collected into table 11.

**Table 10 : simulated distillation conditions**

| | Distillation of the extract | Distillation of the raffinate |
|---|---|---|
| Pressure | 0.15 bar | 0.2 bar |
| Number of stages of the distillation column | 30 | 30 |
| Stage at which the feed is introduced | 15 | 15 |
| Reflux ratio | 0.4 | 1.15 |
| Q-reboiler | 79.09 °C | 54.11 °C |
| | 0.70 MJ/h | 0.21 MJ/h |

**Table 11**

| | **Distillation of the extract** | | **Distillation of the raffinate** | |
|---|---|---|---|---|
| | Distillate 1 | Bottoms 1 | Distillate 2 | Bottoms 2 |
| Pressure | 1 bar | 1 bar | 1 bar | 1 bar |
| Temperature | 25.88 °C | 79.09 °C | 26.08 °C | 54.11 °C |
| rate | 0.9435 kg/h | 0.2406 kg/h | 0.1169 kg/h | 0.6990 kg/h |

| **Composition** | | | | |
|---|---|---|---|---|
| GCOL (Gaïacol) | 0.00 | 4.86 | 0.00 | 0.00 |
| FONE (2(5H)-furanone) | 0.00 | 6.94 | 0.00 | 0.00 |
| ACAC (acetic acid) | 0.52 | 49.46 | 0.00 | 0.00 |
| HONE (hydroxy acetone) | 0.00 | 38.02 | 0.00 | 3.32 |
| H₂O | 5.46 | 0.71 | 3.43 | 96.68 |
| Ethyl acetate | 94.02 | 0.00 | 96.57 | 0.00 |

### Example 4 : Extraction test using a simulated moving bed at 60°C.

The simulation of example 1 has been repeated at a temperature of 60°C. The results are collected in table 12 along with the results of examples 1-3.

**Table 12**

| | Example 1 (SMB at 30°C) | Example 4 (SMB at 60°C) | Example 2 ADD at 60°C (ads. anion exchange resin) | Example 2 ADD at 60°C (Ads. silicalite) | Example 3 Extraction LL |
|---|---|---|---|---|---|
| Feed | Model Mixture | Model mixture | Biomass distillate sample | Biomass distillate sample | Biomass distillate sample |
| Total organic recovery (%) | 94.55 | 95.60 | 76.84 | 93.28 | 89.47 |
| Organics content of the product (wt%) | 99.65 | 99.14 | 99.53 | 99.71 | 99.29 |
| Product composition (wt%) | | | | | |
| GCOL (Gaïacol) | 11.44 | 3.29 | 7.89 | 2.12 | 4.86 |
| FONE (2(5H)-furanone) | 13.67 | 5.84 | 13.13 | 9.51 | 6.94 |
| ACAC (acetic acid) | 37.75 | 43.36 | 66.82 | 50.34 | 49.46 |
| HONE (hydroxy acetone) | 36.79 | 36.79 | 11.69 | 37.74 | 38.02 |
| Total organics | | | | | |
| purity of the solvent recovered(wt%) | >99.99 | >99.99 | 97.49 | 97.55 | 94.30 |
| Estimated Energy Requirements (MJ/kg of product) | 6.75 | 5.83 | 19.22 | 22.76 | 3.776 |

The above simulations demonstrate that the SMB process at 30°C is more efficient in terms of recovery and purity of organic matters and purity of the recovered solvent than liquid-liquid extraction process.

Processes comprising adsorption processes (SMB and ADD) can achieve higher organic recoveries than process including the Liquid-liquid extraction.

The process including the liquid-liquid extraction requires the less energy as energy is only required for distillation.

## Claims

1. Method for producing fuel additives comprising:
(a) a step of providing an aqueous stream comprising organic molecules, said organic molecules including at least carboxylic acids, ketones and aldehydes,
(b) an extraction step in which the aqueous stream provided in step (a) is contacted with at least one solvent, and optionally with at least one adsorbent, and at least a first effluent enriched in organic molecules is recovered, optionally a second effluent depleted in organic molecules is recovered,
(c) a distillation step in which the first effluent of step (b) is submitted to a distillation and at least two streams are recovered: a first solvent stream comprising the solvent(s) and an organic stream comprising the organic molecules,
(d) an optional distillation step in which the second effluent of step (b) is submitted to a distillation step and at least two streams are recovered: a second solvent stream comprising the solvent(s) and an aqueous stream,
(e) a reacting step in which the organic stream of step (c) is contacted with glycerol in the presence of an acid catalyst, the organic molecules containing carboxyl groups are converted into esters by esterification and/or the organic molecules containing carbonyl groups are converted into acetals by acetalization, and an effluent containing the converted organic molecules is produced,
(f) a step of recovery of the effluent produced in step (e) as fuel additive.

2. The method as claimed in claim 1, wherein the aqueous stream provided in step (a) is selected from (i) an aqueous stream formed during pyrolysis of biomass and/or thermal liquefaction of biomass, (ii) an aqueous stream formed during upgrading of biomass oil(s) obtained by pyrolysis of biomass and/or thermal liquefaction of biomass, (iii) an aqueous stream formed during upgrading of a liquid stream containing biomass oil and water, obtained by pyrolysis of biomass and/or thermal liquefaction of biomass, (iv) two or more of the aqueous streams (i), (ii) and (iii).

3. The method as claimed in claim 1 or 2, wherein step (a) includes:
(a1) providing biomass,
(a2) submitting the biomass to pyrolysis or thermal liquefaction and producing (i) a gas phase, (ii) a liquid stream containing oil and water, and (iii) a solid phase,
(a3) separating the liquid stream, and
(a4) recovering from the liquid stream an aqueous stream comprising organic molecules, said organic molecules including at least carboxylic acids, ketones and aldehydes.

4. The method as claimed in any of claims 1 to 3, wherein step (a) includes:
(a5) providing biomass oil(s) or a liquid stream containing biomass oil and water, each obtained from pyrolysis of biomass and/or from thermal liquefaction of biomass,
(a6) upgrading the biomass oil(s) or the liquid stream thereby forming upgraded biomass oil(s) and an aqueous stream comprising organic molecules, said organic molecules including at least carboxylic acids, ketones and aldehydes,
(a7) recovering the aqueous stream.

5. The method as claimed in any of claims 2 to 4, wherein the biomass is selected from lignocellulosic biomass, herbaceous biomass, aquifer biomass, paper, cardboard, organic waste, or mixtures thereof.

6. The method as claimed in any of claims 1 to 5, wherein the extraction step (b) is an adsorption-drying- desorption process including:
(b.1) contacting the aqueous stream with at least one adsorbent adsorbing the organic molecules contained in the aqueous stream,
(b.2) drying the adsorbent,
(b.3) desorbing said at least one adsorbent using at least one solvent and recovering the first effluent enriched in organic molecules.

7. The method as claimed in any of claims 1 to 5, wherein the extraction step (b) is performed in a simulated moving bed device comprising at least three beds of at least one adsorbent, and includes:
(b'.1) introducing separately into the simulated moving bed device the aqueous stream and at least one solvent,
(b'.2) recovering from the simulated moving bed device the first effluent enriched in organic molecules and the second effluent depleted in organic molecules.

8. The method as claimed in any of claims 6 or 7, wherein step (b) comprises one of the following features:
- the solvent used is selected from polar solvents, such as alcohols, ketones, ethers and/or esters, optionally from methanol, ethanol, isopropanol, n-butanol, tert-butanol, ethylene glycol, acetone, methylethylketone, dimethyl ether, ethyl tert-butyl ether, methyl tert-butyl ether, dimethoxymethane, methyl acetate, ethyl acetate or butyl acetate,
- the adsorbent used is selected from (i) polymers having a porous structure, (ii) zeolite-type adsorbents, (iii) clay-based adsorbents, (iv) silica-based adsorbents, (v) oxides, (vii) hydroxides, (viii) oxyhydroxides, (ix) activated carbon-based sorbents, or mixtures of at least two thereof,
- the extraction is performed at a temperature from ambient temperature to 100 °C, preferably up to 80 °C,

9. The method as claimed in any of claims 1 to 5, wherein the extraction step (b) is a liquid-liquid extraction step including:
(b".1) mixing the aqueous stream with at least one solvent non-miscible with water and forming an aqueous phase and an organic phase,
(b".2) separating the aqueous phase from the organic phase, where the organic phase is the first effluent enriched in organic molecules and the aqueous phase is the second effluent depleted in organic molecules.

10. The method as claimed in claim 9 wherein said at least one solvent is selected from ethers, esters furans and aromatic hydrocarbons, optionally selected from butyl acetate, 2-Methyltetrahydrofuran, ethylacetate, methylacetate, ethylformate, methyl formate, isopropyl acetate, n-propyl acetate, ethyl tert-butyl ether, cyclopentyl-methyl ether, tert-amyl-methyl ether, benzene, toluene, xylene, ethylbenzene, phenol, aniline.

11. The method as claimed in any of claims 1 to 10, wherein distillation step (c), and optional distillation step (d), is a vacuum distillation step.

12. The method as claimed in any of claims 1 to 11, wherein the first solvent stream from the distillation step (c), and optionally the second solvent stream from the optional distillation step (d), is reused in the extraction step (b).

13. The method as claimed in any of claims 1 to 12, wherein step (e) comprises one of the following features:
- step (e) is performed at a temperature from ambient temperature to 180 °C,
- step (e) is performed at a atmospheric pressure or under vaccum,
- step (e) is performed for a duration from 15 minutes to 24 hours,
- step (e) is performed while removing water formed,
- step (e) is performed using a homogeneous acid catalyst or a heterogeneous acid catalyst,
- step (e) is performed using an acid catalyst selected from sulphuric acid, 4-methylbenzenesulphonic acid, benzenesulphonic acid, methylsulphonic acid, macroporous styrene-divinylbenzene polymers, preferably comprising an acid functional group such as sulfonic acid, acidic zeolitic-based catalyst, a sulfonated zirconia, acid silica-alumina, ion exchange resins.

14. Installation for producing fuel additives comprising:
- an optional conversion unit (10) for converting biomass (1) into a gaseous phase, a liquid stream containing biomass oil and water, and a solid phase, having an inlet for receiving biomass and at least one outlet from which is recovered the liquid stream,
- an optional separation unit (20) for separating the liquid stream into an organic phase (5) and a first aqueous stream (6), having an inlet receiving the liquid stream, a first outlet from which is recovered the organic phase and a second outlet from which is recovered the first aqueous stream,
- an optional upgrading unit (20) for upgrading the liquid stream or the organic phase into upgraded biomass oil and a second aqueous stream, having an inlet receiving the liquid stream or the organic phase, a first outlet from which is recovered the upgraded biomass oil, a second outlet from which is recovered the second aqueous stream,
- an extraction unit (30) comprising a first inlet receiving the first and/or second aqueous stream (6), a second inlet receiving at least one solvent, at least one outlet from which is recovered a first effluent (8) enriched in organic molecules, and optionally another outlet from which is recovered a second effluent (9) depleted in organic molecules, the extraction unit optionally comprising at least one adsorbent,
- a distillation unit (40) comprising an inlet receiving the first effluent (9) and at least two outlets from which are recovered at least two streams: a first solvent stream (11) comprising the solvent(s) and an organic stream (12) comprising the organic molecules,
- an optional distillation unit (50) comprising an inlet receiving the second effluent (10) and at least two outlets from which are recovered at least two streams: a second solvent stream (13) comprising the solvent(s) and a second aqueous stream (14),
- a reaction unit (60) comprising an inlet receiving the organic stream (12) and at least one outlet from which is recovered as a fuel additive an effluent (16) produced in the reaction unit.

15. Installation as claimed in claim 14, wherein the extraction unit (30) is selected from a liquid-liquid extraction column, a simulated moving bed device, and an adsorption-drying-desorption device.
